# EUROPEAN PATENT APPLICATION

(11) **EP 4 759 238 A1**
(43) Date of publication of application: **17.06.2026**
(21) Application number: 24220303.2
(22) Date of filing: 16.12.2024
(51) Int. Cl.: A61B 6/00, G01T 1/08, H04N 25/76

(54) **AN IMAGING SYSTEM FOR CAPTURING IMAGES OF A SUBJECT DURING X-RAY EXAMINATION**

(71) Applicant: Koninklijke Philips N.V., 5656 AE Eindhoven (NL)
(72) Inventor: VON BERG, Jens, Eindhoven (NL); WEIß, Steffen, Eindhoven (NL); BYSTROV, Daniel, 5656AG Eindhoven (NL); KROENKE-HILLE, Sven, Eindhoven (NL); MENSER, Bernd, Eindhoven (NL)
(74) Representative: Philips Intellectual Property & Standards

(57) **Abstract**

An imaging system is used for capturing images of a subject during X-ray examination. It comprises a video camera system, separate to the X-ray image generation, for generating video camera images. The video camera images are processed to determine that a captured video camera image is synchronized with an X-ray exposure, based on saturation of one or more pixels of the captured video camera image. Timing information can then be generated associating the timing of the video camera images with the timing of the X-ray exposure.

## Description

### FIELD OF THE INVENTION

This invention relates to X-ray imaging, and in particular it relates to the capture of visible light images during an X-ray examination, and the synchronization between the captured images and the X-ray exposure.

### BACKGROUND OF THE INVENTION

Modern X-ray imaging systems typically employ a video camera to observe the acquisition scene. The video camera images may be used for various purposes, such as to obtain respiratory or cardiac information from the patient, or to support the patient positioning or positioning of system parts. Camera images may for example be used for breathing control, auto-collimation, patient motion detection and avoidance, foreign body detection and other quality monitoring and control functions. Video imaging can thus support the examination workflow or the image reading in medical imaging.

This invention relates to approaches which enable the moment of X-ray exposure in a video sequence to be recorded so that images can be identified at the moment of X-ray acquisition. For a proper reading of an X-ray image, it can be helpful to a radiologist to consult a video image at the time of the exposure and/or at a time period directly before or after the exposure.

There are various benefits in identifying images which correspond to the timing of X-ray exposure. Examples are:
to determine the respiratory state of the patient at the time of exposure (by a breathing monitor);
to estimate the cardiac state of the patient at the time of exposure for example by analyzing skin perfusion;
to identify foreign bodies that may appear in the X-ray image and might be confused with disease findings;
to give a visual indication of how to reposition the patient or some equipment visible in the video in case the X-ray taken was not acceptable;
to perform camera-based exposure/dose control;
for saving of camera images for reference purposes in the picture archiving and communication system (PACS).

In each of these examples, it is important to mark the moment of X-ray exposure in the video and to select the corresponding still image (and optionally also image frames some time before and after relative to the exposure moment).

Even if this information is not directly used in the diagnostic chain or in guiding the operator, it may be a useful part of quality control and documentation or it may be useful for training of new algorithms and continuous improvement and learning algorithms based on training or validation pairs of X-rays and synchronized videos.

Identifying video images taken at the time of X-ray exposure requires linking the video stream with the exposure moment. A known way to do this is to synchronize the internal clock of the camera with the internal clock of the X-ray system. This requires a technical interface between the video camera and the X-ray system.

It would be of interest to avoid the need for this interface between the video camera system and the X-ray system, to provide a simpler solution and one that is more easily implemented as a retrofit upgrade to an existing X-ray system.

### SUMMARY OF THE INVENTION

The invention is defined by the claims.

According to examples in accordance with an aspect of the invention, there is provided an imaging system for capturing images of a subject during X-ray examination using an X-ray source and an X-ray detector, the system comprising:
a video camera system, separate to the X-ray detector, for generating video camera images;
a processor for analyzing the video camera images, wherein the processor is configured to:
   determine that a captured video camera image is synchronized with an X-ray exposure generated by the X-ray source based on saturation of one or more pixels of the captured video camera image; and
   generate timing information associating the timing of the video camera images with the timing of the X-ray exposure.

This system provides timing association (i.e. synchronization) between video camera images and an X-ray exposure in a way that does not require any communications interface between the video camera system and an X-ray system (i.e., the X-ray source and X-ray detector). Instead, the camera signal alone is used to determine the moment of X-ray acquisition and relate it to one or more images of the video sequence.

This approach enables a self-sufficient independent video camera system to be used, that derives the timing information from its own sensory data. The solution is independent of the X-ray machine vendor and the specific X-ray machine model or version. It may be a portable solution (e.g. implemented on a tablet or an augmented / mixed reality device). It can be used at different X-ray slots of an X-ray hospital department.

When an X-ray photon reaches the video camera, it typically saturates only one pixel, or just one color channel of the pixel (i.e., one RGB sub-pixel). The X-ray photon leaves the signal level of spatially neighboring pixels untouched, so that a visible image can still be constructed from the non-saturated pixels. It is noted that a "pixel" may be considered to be the smallest independent light detecting element, and hence may comprise a single color (RGB) pixel, namely what may be referred to as a color sub-pixel.

There is a random (stochastic) pattern of the saturated pixels across the image frame, and this pattern also varies stochastically from image frame to image frame. Typically, only a few pixels per frame are affected.

The unaffected pixels provide the correct signal according to the image content. The density of affected pixels is proportional to the output of the X-ray tube.

The video camera system is for example configured such that said one or more pixels are not saturated by visible light. The processor is for example configured to identify pixels that have been saturated and to apply noise reduction based on the identified pixels.

For the noise reduction, the processor is for example configured to process the captured video camera image to reconstruct a pixel signal for the saturated pixels. Spatial interpolation from the non-saturated neighboring pixels may be employed.

In one example, a single video camera is used, both for generating visible light images and for detecting the X-ray exposure. In that case, the "video camera images" are visible light images which function both as "X-ray exposure detection images" and as "visible light images".

Instead, the video camera system may comprise a first video camera which is shieled from visible light, for generating said video camera images, and a second video camera for capturing visible light images. Thus, there is an additional blinded video camera only for detection of the X-ray exposure. One camera is used for X-ray exposure detection (for generating the "X-ray exposure detection images") and the other is used for capturing visible light images (for generating "visible light images"). The two video cameras are synchronized so that timing information is shared between them.

In both examples, the pixels used for X-ray exposure detection are not saturated by visible light, in use.

The processor may be configured to apply a threshold to said one or more pixels to detect the saturation. Furthermore, the processor may be configured to update the threshold over time in dependence on external factors, such as temperature or age of the video camera system.

The video camera system may generate a depth image. It is known to use depth information for the automation and workflow optimization in X-ray imaging. The video camera for example comprises a pair of cameras for generating binocular images.

The video camera system for example comprises one or more CMOS sensors.

The processor may be further configured to identify and process captured images timed with the X-ray exposure and optionally preceding and/or following the X-ray exposure. This image processing enables automated detection of conditions that are relevant to the imaging process.

For example, the processor may be configured to process the captured images to detect foreign bodies. Identified foreign bodies can be marked in the X-ray image when presenting it for diagnosis.

In another example, the processor is configured to process the captured images to monitor a respiratory or cardiac state of the subject.

The respiratory or cardiac state may be used in quantitative diagnosis when assessing cardiac size or lung transparency / opacity. The detection of the cardiac or respiratory state for example is based on the image at X-ray exposure and also images before and after, to determine the phase point in the cardiac or respiratory cycle.

In another example, the processor is configured to process the captured images to monitor patient movement. The pose of the subject after preparation by the radiographer may be compared with the pose at exposure time, so that it can be determined if either improper positioning by the radiographer has caused an insufficient image to be retaken, or a spontaneous movement of the patient in the time between.

The invention also provides an X-ray system comprising:
an X-ray source;
an X-ray detector; and
the imaging system described above.

The X-ray for example comprising a display for displaying an output comprising a combination of an X-ray image and a video camera image from the imaging system. This enables a radiographer to analyze an X-ray image with the benefit of a corresponding visual image.

The invention also provides a method for correlating video camera images of a subject with the timing of X-ray exposure of a subject during X-ray examination using an X-ray source and an X-ray detector, the method comprising:
receiving captured video camera images, captured by a video camera system, separate to the X-ray detector;
analyzing the video camera images to determine that a captured video camera image is synchronized with an X-ray exposure generated by the X-ray source based on saturation of one or more pixels of the captured video camera image; and
generating timing information associating the timing of the video camera images with the timing of the X-ray exposure.

The invention also provides a computer program comprising computer program code means which is adapted, when said program is run on a computer, to implement the method described above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows an X-ray imaging system;
Fig. 2 shows an alternative video camera system; and
Fig. 3 shows a method of correlating video camera images of a subject with the timing of X-ray exposure.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides an imaging system for capturing images of a subject during X-ray examination. It comprises a video camera system, separate to the X-ray image generation, for generating video camera images. The video camera images are processed to determine that a captured video camera image is synchronized with an X-ray exposure, based on saturation of one or more pixels of the captured video camera image. Timing information can then be generated associating the timing of the video camera images with the timing of the X-ray exposure.

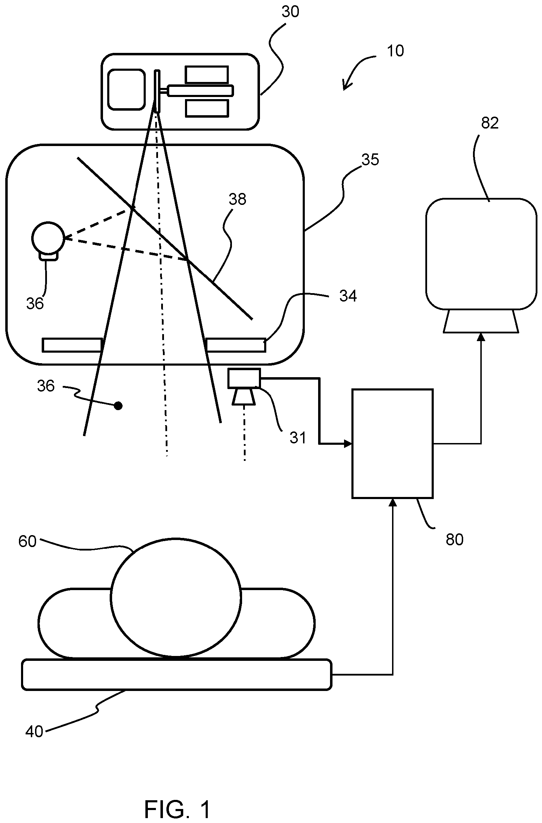

Fig. 1 shows an X-ray imaging system 10, comprising an X-ray source 30 and an X-ray detector 40, between which a body part of a subject 60 is positioned for X-ray imaging.

In the example shown, the view of Fig. 1 is from above, so the subject is standing and the X-ray detector 40 is integrated into a wall stand. This is a typical layout for a chest X-ray. The detector 40 may instead be part of a mobile panel, and a body part such as a hand or foot is placed over the detector, with the X-ray source positioned above.

The X-ray source 30 comprises an X-ray tube, and an X-ray collimator 34 is provided at the output of the X-ray source 30, for generating an X-ray field 36 from the output of the X-ray source 30. The collimator 34 is within a collimator housing 35.

Collimators are used in X-ray detectors to restrict the angles at which X-rays can reach the detector 40. This process minimizes background noise and improves image quality by filtering out scattered rays that can distort the signal. The collimator restricts the X-ray beam to a particular width and height before it reaches the detector, and the collimated area is essentially the footprint of that beam on the detector's surface.

A narrow collimation improves resolution but limits the field of view whereas a broad collimation increases the field of view but may reduce image quality due to scattered radiation.

A visible light source 36 is provided together with optics 38 in the form of a wavelength-selective mirror for directing the visible light source output to the X-ray collimator 34, in order to generate a collimation light field formed of visible light (i.e., in the visible light spectrum). The visible light source is provided, in this example, in the collimator housing 35.

The use of a visible collimation light field is well-known, for example for visually aligning the detector with the irradiation area of the X-ray source. For this purpose, the collimation light field aligns with the X-ray field 36. This visualization is used to align and check the coverage area without directly exposing the area to X-rays, which can be invisible and difficult to measure visually. Thus, collimation light is used for alignment, positioning, and ensuring that the X-ray beam will cover the intended area on the detector.

The visible light source for example comprises a light bulb or laser. The light beam is adjusted to match the X-ray field by using a mirror (as shown), or multiple mirrors and/or lenses that direct it through the same aperture as the X-rays. The light is thus focused to create a narrow, directed beam that mimics the path of the X-ray beam through the collimator's opening. In this way, the light forms an outline of the area that will be irradiated on the detector. For example, it is known to use collimation light to project a visible rectangle, square, or circle (depending on the collimator shape) on the detector or the subject. This projection visually represents the boundaries of the X-ray field.

An operator of the X-ray imaging system may for example adjust the collimator to change the field size of the collimation light beam, which translates to adjusting the X-ray field size.

A video camera system 31 is provided for generating video camera images. It is known to capture visual images to provide additional information to the radiographer. Thus, the radiographer may perform visual inspection of both an X-ray image and a camera image of the patient in order to assist in the interpretation of the X-ray image.

The video camera system may comprise a depth camera, for example with two, binocular, image sensors. The image sensor or image sensors typically comprise CMOS cameras or CCD arrays. The video camera system 31 is for example mounted outside the collimator housing 35 facing the detector.

This invention is based on the recognition that the video camera system is also able to detect the X-ray radiation released from the X-ray tube at the exposure time.

The ability of CMOS sensors to detect X-ray radiation has been reported, for example in Kang, Han Gyu, et al. "An investigation of medical radiation detection using CMOS image sensors in smartphones." Nuclear Instruments and Methods in Physics Research Section A: Accelerators, Spectrometers, Detectors and Associated Equipment 823 (2016): 126-134.

In the configuration shown in Fig. 1, the video camera system 31 is exposed to scatter radiation or back scatter.

When a scattered or back scattered X-ray photon reaches the video camera, it typically saturates a pixel, in particular just one color channel of the pixel (i.e., one RGB sub-pixel). Thus, it affects only the smallest detection element of the sensor array, which will be termed a "pixel" in this disclosure. The X-ray photon leaves the signal level of spatially neighboring pixels untouched, so that a visible image can still be constructed from the non-saturated pixels.

There is a random (stochastic) pattern of the saturated pixels across an image frame, and this pattern also varies stochastically from image frame to image frame. Typically, only a few pixels per frame are affected.

This invention is based on the recognition that the saturation of camera pixels by scattered X-ray radiation can be used to detect the timing of the X-ray exposure, so that synchronization can be provided between independently captured X-ray images and visible light images.

Fig. 1 shows a processor 80 for analyzing the video camera images to perform the required timing analysis.

The processor determines that a captured video camera image is synchronized with an X-ray exposure generated by the X-ray source based on saturation of one or more pixels of the captured video camera image. In this way, the image frame which corresponds to the timing of the X-ray exposure can be tagged as having a timing of the X-ray exposure, and the other images of the video sequence can be tagged with timing information relative to the X-ray exposure time. More generally, timing information is generated associating the timing of the video camera images with the timing of the X-ray exposure.

The X-ray exposure may have a duration which extends to two or more video image frames.

The video stream is for example divided into a still image corresponding to the X-ray exposure timepoint, a video steam before and a video stream after.

In this way, synchronization is enabled between the video camera images and an X-ray exposure in a way that does not require any communications interface between the video camera system and the X-ray system. Instead, the camera signal alone is used to determine the moment of X-ray acquisition and relate it to one or more images of the video sequence.

In a first approach, the video camera system comprises a single camera (or a depth camera) which is used both to generate visible images and to detect X-ray exposure timing. The saturated pixels may be considered to create an "X-ray exposure detection image" and the non-saturated pixels may be considered to create "visible light images".

In this approach, the non-saturated pixels are used to generate an image. To enable pixel saturation to reliably indicate exposure to an X-ray photon, the pixels of the video camera system are not saturated by visible light (i.e. by the intensity of visible light to be experienced in normal operating conditions of the system).

After identifying pixels that have been saturated, noise reduction can then be applied to enable generation of the visible image. For example, a non-saturated pixel signal may be reconstructed for the saturated pixels. Spatial interpolation from the non-saturated neighboring pixels may be employed.

The processor may detect saturation by applying a threshold to the signal level detected by the individual pixels. Furthermore, the threshold may be calibrated to the system and it may also be updated over time in dependence on external factors, such as temperature or age of the video camera system.

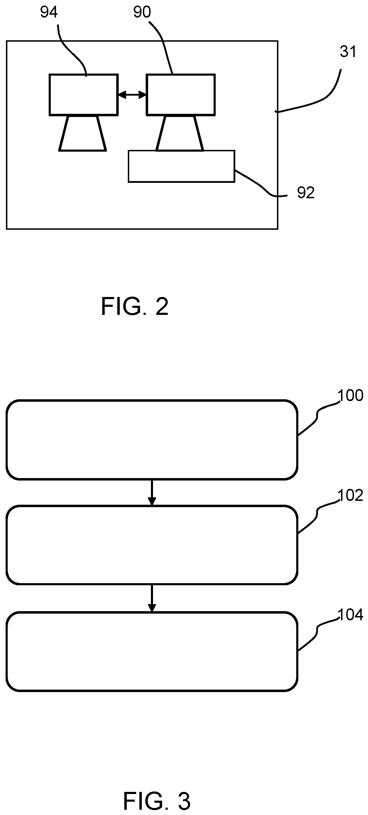

In a second approach, as shown in Fig. 2, the video camera system 31 comprises a first video camera 90 which is shielded from visible light by a filter 92, for generating the "X-ray exposure detection images" and a second video camera 94 for capturing the "visible light images". Thus, an additional blinded video camera 90 may be used only for detection of the X-ray exposure. The two video cameras are synchronized so that timing information is shared between them. Thus, detection of the timing of the X-ray exposure from the X-ray exposure detection image is duplicated to the visible light image.

The second video camera may then be shieled from the X-rays by a filter so that the pixels will not be saturated by the scattered or back scattered X-rays.

As mentioned above, the video camera system may generate a depth image. It is known to use depth information for the automation and workflow optimization in X-ray imaging. For example, it is known to use depth information to assist in accurately positioning the patient, for example by capturing a 3D contour of their body. This information can be used to ensure that the body part being imaged is in the correct orientation and distance relative to the X-ray source and detector, minimizing errors caused by misalignment. This helps to reduce the need for repeat scans due to incorrect positioning. By analyzing the patient's body contours, a depth camera can also help adjust X-ray parameters (such as intensity) based on the thickness or depth of the area being imaged.

Depth cameras can also support automation in digital radiography by integrating with imaging software. For example, by identifying the patient's body position the X-ray system may be adjusted automatically. Depth cameras can also track patient movement in real-time, ensuring that the patient remains in the correct position throughout the imaging process.

For depth imaging, the video camera for example comprises a pair of cameras for generating binocular images. The presence of two cameras also provides redundance. By having two cameras observing the same scene, there will be two detector pixels associated with each scene location. An X-ray event can be detected with high probability, based on one pixel being saturated even when the other is not.

The processor may perform image analysis. For example, at least the still image may be analyzed, but analysis of the video stream before and after X-ray exposure may also be of intertest.

A first example of image analysis is to detect foreign bodies. Identified foreign bodies can be marked in the X-ray image when presenting it for diagnosis. The foreign bodies are in this way automatically related to signs in the X-ray image. Foreign bodies visible in the image at the time of X-ray exposure may be most relevant (since it represents the exact scene as captured in the X-ray image). However, some foreign bodies may however be more easily identifiable in the video before or afterwards where they present themselves differently.

A second example of image analysis is to monitor a respiratory or cardiac state of the subject. The respiratory or cardiac state may be used in a quantitative diagnosis when assessing cardiac size or lung transparency / opacities. The detection of the cardiac or respiratory state may be based on analysis of the still image at the X-ray exposure time and also the video stream before and after in order to determine the phase point in the cardiac or respiratory cycle.

A third example of image analysis is to monitor movement. The video camera system may perform motion monitoring, by comparing the pose of the subject after preparation by the radiographer with the pose at the exposure time , to indicate if either improper positioning by the radiographer has caused an insufficient image to be retaken, or a spontaneous movement of the patient in the time between.

Fig. 1 shows a display 82 for displaying an output comprising a combination of an X-ray image and a video camera image from the imaging system. This enables a radiographer to analyze an X-ray image with the benefit of a corresponding visual image. Images may be presented side-by-side or superposed, or a hybrid image may be formed by combining features from the X-ray image and the visible light image.

For example, quality issues in the X-ray image can be understood by referring to the video image. The images may for example be spatially aligned to provide information on how to correct the setting of patient and equipment next time, e.g., a leg has to be rotated inwards to better image the ankle joint.

It is described above how the saturation analysis provides X-ray exposure timing. In addition, the total dose may be also estimated for quality control. The number of saturated pixels depends on the dose, so with suitable calibration, the determination of the number (and possibly also pattern characteristics) of the saturated pixels can also be translated to a dose level.

Fig. 3 shows a method for correlating video camera images of a subject with the timing of X-ray exposure of a subject during X-ray examination using an X-ray source and an X-ray detector. The method comprises:
In step 100, receiving captured video camera images, captured by a video camera system separate to the X-ray detector;
In step 102, analyzing the video camera images to determine that a captured video camera image is synchronized with an X-ray exposure generated by the X-ray source based on saturation of one or more pixels of the captured video camera image; and
In step 104, generating timing information associating the timing of the video camera images with the timing of the X-ray exposure.

The invention has been described above with reference to digital radiography X-ray systems. However, the invention may be applied to other X-ray imaging systems, such as CT scanners.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. An imaging system for capturing images of a subject during X-ray examination using an X-ray source (30) and an X-ray detector (40), the system comprising:
a video camera system (31), separate to the X-ray detector, for generating video camera images;
a processor (80) for analyzing the video camera images, wherein the processor is configured to:
determine that a captured video camera image is synchronized with an X-ray exposure generated by the X-ray source based on saturation of one or more pixels of the captured video camera image; and
generate timing information associating the timing of the video camera images with the timing of the X-ray exposure.

2. The imaging system of claim 1, wherein the video camera system (31) is configured such that said one or more pixels are not saturated by visible light.

3. The imaging system of claim 1 or 2, wherein the processor (80) is configured to process the captured video camera image to reconstruct a pixel signal for the saturated pixels.

4. The imaging system of claim 1 or 2, wherein the video camera system (80) comprises a first video camera (90) which is shieled from visible light, for generating said video camera images, and a second video camera (94) for capturing visible light images.

5. The imaging system of any one of claims 1 to 4, wherein the processor (80) is configured to apply a threshold to said said one or more pixels to detect the saturation.

6. The imaging system of claim 5, wherein the processor (80) is configured to update the threshold over time, in dependence on external factors, such as temperature or age of the video camera system.

7. The imaging system of any one of claims 1 to 6, wherein the video camera system (31) generates a depth image.

8. The imaging system of any one of claims 1 to 7, wherein the video camera system (31) comprises a CMOS sensor.

9. The imaging system of any one of claims 1 to 8, wherein the processor (80) is further configured to identify and process captured images timed with the X-ray exposure and optionally preceding and/or following the X-ray exposure.

10. The imaging system of claim 9, wherein the processor (80) is configured to process the captured images to detect foreign bodies.

11. The imaging system of claim 8 or 9, wherein the processor (80) is configured to process the captured images to:
monitor a respiratory or cardiac state of the subject; or
monitor movement of the subject.

12. An X-ray system comprising:
an X-ray source (30);
an X-ray detector (40); and
the imaging system of any one of claims 1 to 11.

13. The X-ray system of claim 12, comprising a display (82) for displaying an output comprising a combination of an X-ray image and a video camera image from the imaging system.

14. A method for correlating video camera images of a subject with the timing of X-ray exposure of a subject during X-ray examination using an X-ray source and an X-ray detector, the method comprising:
(100) receiving captured video camera images, captured by a video camera system separate to the X-ray detector;
(102) analyzing the video camera images to determine that a captured video camera image is synchronized with an X-ray exposure generated by the X-ray source based on saturation of one or more pixels of the captured video camera image; and
(104) generating timing information associating the timing of the video camera images with the timing of the X-ray exposure.

15. A computer program comprising computer program code means which is adapted, when said program is run on a computer, to implement the method of claim 14.
